# EUROPEAN PATENT APPLICATION

(11) **EP 1 884 567 A1**
(43) Date of publication of application: **06.02.2008**
(21) Application number: 06016322.7
(22) Date of filing: 04.08.2006
(51) Int. Cl.: C12N 9/24, C12N 9/78, C12P 19/26, C12N 1/21, C12N 1/15

(54) **Nucleic acid molecules encoding a bacterial protein having deacetylase activity, such protein, methods for producing same, and uses thereof**

(71) Applicant: Institute of Molecular Biology and Biotechnology, 711 10 Heraklion (GR); Moerschbacher, Bruno, Prof. Dr., 48161 Münster (DE)
(72) Inventor: Bouriotis, Vassilis, Prof. Dr., 71003 Heraclion Crete (GR); Deli, Alexandra, 71307 Heraclion Crete (GR); Psylinakis, Emmanuel Department of Human Nutrition and Dietetics, 72300 Siteia Crete (GR)
(74) Representative: Leifert & Steffan

(57) **Abstract**

The invention relates to nucleic acid molecules encoding a bacterial protein having deacetylase activity, to the protein itself, methods for producing of the protein and different uses thereof. In particular the invention relates to the BC1534 deacetylase protein its production and use for the production of chitosan and/or N-acetylglucosamine.

## Description

The present application is related to nucleic acid molecules encoding a bacterial protein having deacetylase activity, to such protein iself, to methods for producing such protein, and to different uses thereof.

Said protein having deacetylase activity may be used for the production of chitosan, a polysaccharide which due to its particular features has attracted worldwide attention, one of these features being that it is one of the few biopolymers having a positive net charge under physiological conditions.

This feature is responsible for the fact that chitosan interacts with many polyanionic polymers such as proteins, DNA, fatty acids, fat molecules and lipids. This physicochemical interaction is assumed to account for some of the biological activities of chitosans.

Chitosan is a linear co-polymer of glucosamine (2-desoxy-2-amino-β-D-glucopyranose, GlcN) and N-acetylglucosamine (2*-desoxy-2-acetamido-β-D-glucopyranose, GlcNAc) in β-1,4-glycosidic linkages. The content of GlcNAc (and thus the degree of acetylation of the polymer) varies widely.

Chitosan is not very common in nature. It is mainly found in the cell walls of a small group of fungi called Zygomycetes. Chitosan for commercial purposes is made from chitin, which is a very widely distributed biopolymer, being found in most fungal cell walls and the exoskeleton of most arthropods.

The latter is usually isolated from shrimp and crab shells, which are cheaply available as being remnants of the fastly growing crab- and shrimp aquaculture production. The two step isolation process involves demineralisation (elimination of calcium carbonate, which represents around 30 % (w/w) of the cuticle) by treatment with an excess of HCl (0.25-1 %, 12-24 h, room temperature) followed by washing steps, and deproteination by repeated treatment with an excess of hot aqueous NaOH (0.25-1 M, 1-3 h, 60-70°C) followed by washing steps, neutralisation, and drying.

The thus obtained chitin is then modified by deacetylation of part of its GlcNAc-residues. This is usually achieved by repeated incubation of chitin in an excess of concentrated hot alkali, mainly NaOH (35-50 %, 1-3 h, 90-130 °C), followed by neutralisation, washing steps, and drying. However, this procedure leads to inhomogeneous random loss of acetyl groups, and to a partial depolymerisation of the polymer. Moreover, some producers add a final bleaching step by means of various oxidising treatments (UV, O₃, H₂O₂). The chitosan thus obtained is highly deacetylated, with degrees of acetylation ranging from 0 to 20 %.

These chitosan preparations, if not further purified, contain insoluble, highly N-acetylated fractions that originate from the core of the chitin granules submitted to heterogeneous chemical deacetylation. The N-acetyl-groups in the acid-soluble fractions are randomly distributed due to the random chemical deacetylation procedure.

The quality of the produced chitosan varies greatly, as even other bonds within the chitin polymer are cleaved by the above methods as well, leading to a very heterogeneous product.

This relatively cheap bulk chitosan is therefore characterised by high variability and heterogeneity in terms of both the degree of polymerisation and the degree of N-acetylation. The obtained product is however suitable for a variety of applications, such as waste water treatment.

More demanding applications, however, require better defined chitosans which are not available today. For these reasons, a need for more specific methods to produce well-defined chitosans exists.

Tanaka et al. (J Biol Chem. 2004; 279: 30021-7) have described a diacetylchitobiose deacetylase from the hyperthermophilic archaebacteria *Thermococcus kodakaraensis.* The recombinant deacetylase protein showed deacetylase activity toward N-acetylchitooligosaccharides, and the deacetylation site was revealed to be specific at the nonreducing GlcNAc residue. The enzyme also deacetylated GlcNAc monomers. The deduced amino acid sequence was classified into the LmbE protein family, which includes N-acetylglucosaminylphosphatidylinositol de-N-acetylases and 1-D-myo-inosityl-2-acetamido-2-deoxy-alpha-D-glucopyranoside deacetylases as well.

The *Thermococcus* deacetylase was found to be suitable for the production of chitosan from chitin, either by deacetylation of GlcNac-residues in a GlcNAc-Oligomer, a GlcNAc-Polymer or in a chitin molecule, or by deacetylation of GlcNAc-monomers and subsequent polymerization with untreated GlcNAc-monomers, respectively.

However, the expression yield of this protein is relatively poor, so that the expressed protein can only be produced at low rates, making it expensive and thus not very attractive for commercial applications. Moreover, the protein develops its maximum activity at 75° C and pH 8.5. These conditions are not appropriate for certain applications.

It is thus one objective of the present invention to provide a deacetylase enzyme having deacetylase activity towards GlcNAc-molecules, which can be produced more cheaply and which is thus better suited for industrial applications of the above mentioned kind.

It is another objective of the present invention to provide a method for producing chitosan from chitin which is more economical than the above described method and/or provides better defined chitosans.

It is a further objective of the present application to provide the amino acid sequence and the respective nucleic acid sequence for said protein.

These and other objectives will become evident to those skilled in the art from the specification. The objectives are solved by the independent claims of the present invention.

According to the invention, a nucleic acid molecule is provided which is selected from the group consisting of :
a) nucleic acid molecules encoding a mature form of the polypeptide having the deduced amino acid sequence as set forth in SEQ ID No: 2, said polypeptide having deacetylase activity;
b) nucleic acid molecules comprising the nucleotide sequence as set forth in SEQ ID No: 1, and which encode for a mature form of the said polypeptide;
c) nucleic acid molecules encoding a fragment or derivative of a polypeptide encoded by a polynucleotide of any one of a) to b), wherein in said derivative one or more amino acid residues are conservatively substituted compared to said polypeptide, and said fragment or derivative has deacetylase activity;
d) nucleic acid molecules the complementary strand of which hybridizes under stringent conditions to a nucleic acid molecule as defined in any one of a) to c), and which encode a protein having deacetylase activity;
e) nucleic acid molecules which are at least 95 % identical to a nucleic acid molecule as defined in any one of a) to d) and which encode a protein having deacetylase activity;
f) nucleic acid molecules which are at least 70 % identical to a nucleic acid molecule as definded in any one of a) to d) and which encode a protein having deacetylase activity;
g) or the complementary strand of such nucleic acid molecule.

The encoded protein having deacetylase activity is the BC1534 deacetylase protein which is derived from *Bacillus cereus*. Among these, a preferred cell line is *Bacillus cereus* ATCC 14579.

It is further provided a method for producing a protein having deacetylase activity, comprising the steps of:
a) cloning a nucleic acid molecule according to claim 1 into an expression vector,
b) transferring the expression vector into an expression system,
c) incubating the expression system,
d) inducing the expression of the modified expression vector,
d) purifying the expressed protein having deacetylase activity.

In this method, the expression system is preferably a prokaryotic cell. Eukaryotic cells, i.e. yeast cells, plant cells or animal cells may however be used as well.

The transfection may be accomplished by electroporation or chemical transformation. For the former, an electric shock of 2.5 kV, 25 µF, 200 Ohms and 5 ms may be applied. For the latter, CaCl₂ and a TSS-solution containing Dimethylsulfoxide may be applied. However, any other transfection method known in the state of the art may also be used.

Prior to transfection the cells have to be made competent, i.e. turned into a state in which they are susceptible to the transfection. This may for example be accomplished by harvesting cells which are in the logarithmic growth phase. These cells are then washed with an ice-cold Water-Glycerol mixture.

In a preferred embodiment of this method the produced protein is the BC1534 deacetylase protein.

Any suitable expression plasmid may be used as the expression vector of the above method. Preferably, the plasmid pET26b is used. pET26b is supplied by Novagen. The plasmid comprises a T7 lac promoter which enables induction of protein expression by standard means. The plasmid has a backbone size of 5400 base pairs, and it comprises a multiple cloning site and a Kanamycin resistance, moreover.

In another preferred embodiment of the above method, the *E*. *coli* strain BL21-DE3 is used as the expression system. The combination of *E. coli* strain BL21-DE3 and pET26b is especially advantageous, as both have been designed to work well with each other.

The expression of the vector is preferably induced by addition of isopropyl β-D-thiogalactopyranoside (IPTG). As pET26b contains a T7 lac promotor, IPTG is well suited for this purpose. If, however, an expression vector comprising a different promotor is used, a different induction agent will be used. State of the art provides a variety of promoter/induction agent-pairs, which may all be used in the method of the invention.

In another preferred embodiment, the expression vector comprises a tag for subsequent protein purification. Preferably, this tag is a His(6)-tag at the C-terminus of the vector, as already being comprised in the pET26b plasmid. Other tags known in the art, such as a GST-tag, may however be used as well.

Purification of the expressed protein having deacetylase activity is preferably carried out by affinity chromatography and/or gel filtration chromatography. Among these methods, immobilized metal affinity chromatography (IMAC) is especially preferred, particularly in connection with the above mentioned His (6) -tag.

Furthermore, an expression vector containing one of the above recited nucleic acid molecules is provided. This vector is for example the pET26b plasmid into which the respective nucleic acid molecule was cloned. Any other suitable plasmid from the state of the art may also be used to produce the expression vector of the invention. Other suitable exporession vectors may contain cosmids or YACs.

Yet another object of the invention is to provide a host cell transformed with said nucleic acid molecule or with said expression vector, wherein said transformed cell serves as an expression system. As stated above, a prokaryotic cell is preferably used for that purpose.

Other suitable host cells include yeast cells, plant cells or animal cells including insect cells and immortalized cell lines. These cells require different expression systems, which are known in the art *per se*.

For example, viral expression systems including recombinant bacteriophages, baculoviruses, adenoviruses, or lentiviruses my be used. Transfecting eukaryotes including *Agrobacterium tumefaciens* bearing a recombinant Ti-plasmid may as well be used.

Furthermore, a protein having a deacetylase activity is provided by the invention, wherein the protein is encoded by said nucleic acid molecule, or obtainable by the above recited method. This protein is the BC1534 protein from *Bacillus cereus.*

The invention does moreover provide different methods for producing chitosan, in which the above protein is applied. In one embodiment, GlcNAc-residues in a GlcNAc-Oligomer, a GlcNAc-Polymer or in a chitin molecule are deacetylated with said protein.

The deacetylation of individual GlcNAc-residues provides a molecule comprising both GlcN- and GlcNAc-residues. The degree of acetylation and the acetylation pattern of the resulting polymer are governed by the reaction conditions, i.e. temperature, pressure, duration, use of particular reaction matrices, and the like.

In another embodiment, G1cNAc-monomers are deacetylated with said protein, and the resulting G1cN-monomers are then polymerized with untreated G1cNAc-monomers in order to obtain chitosan. In this method, the degree of acetylation and the acetylation pattern of the resulting polymer can be accurately defined by adjusting the reaction conditions of the synthesis reaction. Accordingly, the invention provides a chitosan produced by any of these two methods.

For example due to their positive net charge, chitosans are suitable for a variety of applications for example in biotechnology, chemistry, cosmetics, agriculture, food technology, medicine, engineering & environmental technology. Almost daily, new and promising fields of application are found.

One further object of the invention is thus the use of the chitosan produced with the above method for such purposes. These applications do greatly benefit from the fact that the method of the present invention provides high quality chitosans, i.e. chitosans with a well-defined degree of acetylation, a well-defined acetylation pattern, and so forth.

Applications according to the invention include for example the use for the immobilization of enzymes and cells in biotechnology research and procedures, for the strengthening and/or coloring of human and/or animal hair, or the use as skin and/or nail care.

Other applications include the use as seed and/or crop protection and/or preservation agent, the use for the adsorption of endotoxins and nucleic acids, or the use in paper processing.

The chitosan of the above method may moreover be used as a polymer carrier for catalysts or an intermediate product for the synthesis of fine chemicals. In agriculture, the chitosan may be used as an additive for whey feed or as a composting accelerator. In food technology, the chitosan may serve a biodegradable packing material, as a clarifying means for juices, as a flocculation agent, as dietary fibre or as a food coating.

A very promising yet demanding application is the use as a dietetic additive for weight loss. Due to its positive net charge chitosan is supposed to bind fatty acids and reduce the fat uptake in the intestines.

Medical applications lie in the field of wound dressing, surgical seam material, contact lenses material and nanoparticles for drug, gene or vaccine delivery.

The chitosan of the invention may moreover be used as loudspeaker diaphragm material, embedding medium for electron microscopy, or precipitant for waste water purification.

In another embodiment of the present invention the protein having deacetylase activity according to the present invention is employed in a reverse hydrolysis reaction to produce N-acetylglucosamine from glucosamine. This reverse or backward reaction is possible and desirable since large amounts of N-acetylglucosamine in high quality are needed for industrial applications. The backward reaction can be carried out in analogy to Tokuyasu et al., Carbohydrate Research 322 (1999) 26-31.

### Examples and Figures

The following examples and figures are presented to illustrate the present invention and are not to be construed to limit the scope of the appended claims in any manner whatsoever. Many variations of the present invention will suggest themselves to those skilled in the art in light of the above-detailed description. All such modifications are within the full-intended scope of the claims.

### Example

### Bacterial Strains, Plasmids, and Medium

*B*. *cereus* ATCC 14579 was grown aerobically 30°C in standard nutriuent broth (LB Medium) containing collodoial chitin and/or GlcNAc-dimers as induction agent. Induction of bc1534 gene expression was observed after 12 hrs or 24 hrs, respectively.

The pET26b vector was used for cloning the bc1534 gene, and the *E. coli* strain BL21-DE3 was used as a host for the expression vector. Both BL21-DE3 and pET26b were obtained from Novagen, Madison, WI.

### Cloning procedure

The bc1534 gene was cloned into the pET26b vector with a His(6)-tag at its C-terminus. For this purpose, the bc1534 gene was digested with the *NdeI* and *XhoI* restriction endonucleases and ligated into the pET26b vector. The stop codon TTA had before been removed, so that the later protein would bear a His(6)-tag at its C-terminus.

The bc1534/pET26b construct was transferred into electrocompetent BL21-DE3 cells by electroporation according to standard protocols. The transfected cells were cultivated in LB medium at 30 °C overnight. The following day, the culture was diluted with 50 parts LB medium, and, when an OD₆₀₀ of 0.6 was achieved, the culture was induced by addition of 1mM IPTG and cultivated at 30°C overnight.

### Purification of the protein

The bacterial pellet was homogenized in a buffer containing 25 mM Tris-HCl, 300 mM NaCl and 1mM PMSF, and further sonicated. The lysate was then centrifuged, and the supernatant was loaded onto a Ni-NTA column after a 1/3 dilution with the previous buffer. After an extended wash with the same buffer, the protein was eluted with a buffer containing 150 - 200 mM imidazole. Despite the high purity of the protein, a gel filtration chromatography was carried out thereafter. The isolated enzyme had a molecular weight of approx. 30 kDa.

### Yield and enzyme activity

A yield of 10 mg enzyme was obtained from 1000 ml of *E*. *coli* culture. This is a very high yield compared to a previously known deacetylase from *Thermococcus kodakaraensis* (Tanaka et al. (J Biol Chem. 2004; 279: 30021-7), the gene of which shares 31 % sequence identity with bc1534, and which belongs to the same uncharacterized family of LmbE-like proteins.

An enzymatic assay was used to evaluate the activity of the enzyme. The assay was based on three coupled reactions, wherein the amount of the acetic acid released in the last reaction was measured. The reaction buffer used contained 50 mM HEPES-NaOH and 1 mM CoCl₂. The latter was necessary for the enzyme to be active. A pH of 8,0 and a temperature of 37°c were established.

With this assay, a specific activity of 0.03 U mg⁻¹ for GlcNAc was determined at 37 °C. The enzyme deacylated (GlcNAc)₂ and (GlcNAc)₃ as well, but the activity was lower for these substrates.

In contrast to this, the above described deacetylase from *Thermococcus kodakaraensis* was reported to only partially deacylate GlcNAc-Oligomers. The maximum enzyme activity reported was 0.0167 U mg⁻¹ at 70 °C. The activity at 37 °C was even lower by 20%.

### Figures

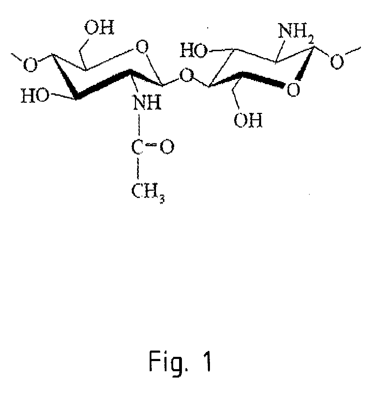

Referring to Fig. 1, the idealized structure of chitosan is shown, as demonstrated by a dimeric residue consisting of glucosamine (2-desoxy-2-amino-β-D-glucopyranose, GlcN, right molecule) and N-acetylglucosamine (2-desoxy-2-acetamido-β-D-glucopyranose, GlcNAc, left molecule) in β-1,4-glycosidic linkages. As can be derived from the above description, the degree of acetlyation (i.e. the share of GlcNAc in the chitosan polymer) varies within certain borders.

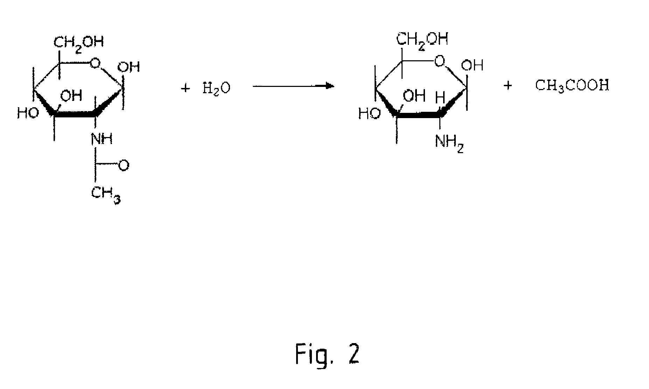

Referring to Fig. 2, the deacetylation reaction of a GlcNAc molecule as catalyzed by the BC 1534 deacetylaase enzyme of the present invention is shown. Acetic acid is released as a reaction product. The specific activity of the BC 1534 protein of the present invention is 0.03 U mg⁻¹ for GlcNAc-monomers. The temperature optimum is at about 37 °C, and the pH optimum is at pH 8.0.

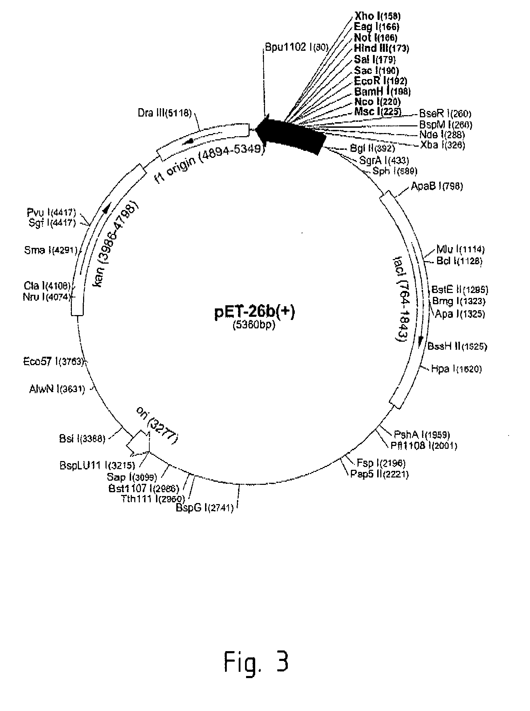

Referring to Fig. 3, the plasmid pET-26b(+) as provided by Novagen is shown. Attention is drawn to the multiple cloning site and the *lacI*-gene under control of a T7 promotor.

## Claims

1. A nucleic acid molecule selected from the group consisting of:
a) nucleic acid molecules encoding a mature form of the polypeptide having the deduced amino acid sequence as set forth in SEQ ID No: 2, said polypeptide having deacetylase activity;
b) nucleic acid molecules comprising the nucleotide sequence as set forth in SEQ ID No: 1, and which encode for a mature form of the said polypeptide;
c) nucleic acid molecules encoding a fragment or derivative of a polypeptide encoded by a polynucleotide of any one of a) to b), wherein in said derivative one or more amino acid residues are conservatively substituted compared to said polypeptide, and said fragment or derivative has deacetylase activity;
d) nucleic acid molecules the complementary strand of which hybridizes under stringent conditions to a nucleic acid molecule as definded in any one of a) to c), and which encode a protein having deacetylase activity;
e) nucleic acid molecules which are at least 95 % identical to a nucleic acid molecule as defined in any one of a) to d) and which encode a protein having deacetylase activity;
f) nucleic acid molecules which are at least 70 % identical to a nucleic acid molecule as definded in any one of a) to d) and which encode a protein having deacetylase activity;
g) or the complementary strand of such nucleic acid molecule.

2. The nucleic acid according to claim 1, in which the encoded protein having deacetylase activity is the BC1534 deacetylase protein.

3. A method for producing a protein having deacetylase activity, comprising the steps of:
a) cloning a nucleic acid molecule according to claim 1 into an expression vector,
b) transferring the expression vector into an expression system,
c) incubating the expression system,
d) inducing the expression of the modified expression vector,
d) purifying the expressed protein having deacetylase activity.

4. The method according to claim 3, wherein the produced protein is the BC1534 deacetylase protein.

5. The method according to any of claims 3 - 4, wherein the expression vector is the plasmid pET26b.

6. The method according to any of claims 3 - 5, wherein the expression system is the *E. coli* strain BL21-DE3.

7. The method according to any of claims 3 - 6, wherein the expression of the vector is induced by addition of isopropyl β-D-thiogalactopyranoside.

8. The method according to any of claims 3 - 7, wherein the expression vector comprises a tag for subsequent protein purification

9. The method according to any of claims 3 - 8, wherein the expressed protein having deacetylase activity is purified by affinity chromatography and/or gel filtration chromatography.

10. An expression vector containing the nucleic acid molecule of claims 1 or 2.

11. A host cell transformed with the nucleic acid molecule of claim 1 or with the expression vector of claim 10, said cell serving as an expression system.

12. A protein encoded by the nucleic acid molecule of claim 1 or 2, or obtainable by the method of claim 3, said protein having a deacetylase activity.

13. The protein of claim 12, said protein being the BC1534 deacetylase protein.

14. A method for producing chitosan from chitin, wherein N-acetylglucosamin-residues (GlcNAc) in a GlcNAc-Oligomer, a GlcNAc-Polymer or in a chitin molecule are deacetylated with a protein according to claims 12 or 13.

15. A method for producing chitosan, wherein GlcNAc-monomers are deacetylated with a protein according to claims 12 or 13 in order to produce glucosamin (GlcN), which are then polymerized with untreated GlcNAc-monomers in order to obtain chitosan.

16. A chitosan produced with a method according to claims 14 or 15.

17. Use of a chitosan according to claim 15 for a purpose selected from the group consisting of:
a) immobilization of enzymes and cells in biotechnology research and procedures;
b) strengthening and/or coloring of human and/or animal hair
c) skin and/or nail care;
d) seed and/or crop protection and/or preservation;
e) adsorption of endotoxins and nucleic acids; or
f) paper processing.

18. Use of a chitosan according to claim 15 as
a) a polymer carrier for catalysts;
b) an intermediate product for the synthesis of fine chemicals;
c) an additive for whey feed;
d) a composting accelerator;
e) packing material;
f) a clarifying means for juices;
g) a flocculation agent;
h) dietary fibre for foodstuff;
i) food coating;
j) wound dressing;
k) surgical seam material;
l) a dietetic additive for weight loss;
m) contact lenses;
n) nanoparticles for drug, gene or vaccine delivery;
o) a loudspeaker diaphragm;
p) an embedding medium for electron microscopy; or
q) a precipitant for waste water purification.

19. A method for producing N-acetylglucosamine from glucosamine wherein a protein according to claim 12 or 13 is used as a catalyst.

20. Use of a protein according to claim 12 or 13 as a catalyst in the production of N-acetylglucosamine from glucosamine.


SEQUENCE LISTING

<110> University of Crete
      University of Crete
      Bouriotis, Vassilis

<120> BC1534 deacetylase

<130> S05830

<160> 2

<170> PatentIn version 3.3

<210> 1
<211> 750
<212> DNA
<213> Bacillus cereus

<400> 1
atgagtggat tacatatatt agcgtttggt gctcatgccg atgatgttga aatcggcatg 60
gctggtacca tcgctaaata tacaaagcaa gggtatgaag taggtatttg tgatttaaca 120
gaagctgatc tttcttcaaa tggaacgata gagttgagaa aagaagaagc gaaggtcgca 180
gctcgtataa tgggagtaaa aacgaggctg aatttagcga tgccagaccg tggtttgtat 240
atgaaagaag agtatatacg tgaaattgtc aaggtgatcc gtacatataa accaaaacta 300
gtttttgcgc cgtactatga agatcgccat ccagatcatg caaattgtgc aaagcttgtg 360
gaagaggcta ttttttcagc aggaatccgt aaatatatgc cggaactttc accacatcgt 420
gtggagtctt tttataatta tatgattaat ggttttcata aaccgaattt ttgtatagat 480
ataagtgagt acctctctat aaaggtggaa gcattagaag cgtatgaaag tcagttttca 540
acagggagtg atggtgttaa gacaccgtta acagaaggtt acgttgaaac tgtgattgct 600
cgtgagaaga tgtttggaaa agaagttgga gtcttgtatg ccgagggatt tatgagtaag 660
aaaccggttt tattacatgc tgatttatta gggggatgta aatgaaaacc ggttttatta 720
catgctgatt tattaggggg atgtaaatga 750

<210> 2
<211> 234
<212> PRT
<213> Bacillus cereus

<400> 2

Met Ser Gly Leu His Ile Leu Ala Phe Gly Ala His Ala Asp Asp Val
1               5                   10                  15
Glu Ile Gly Met Ala Gly Thr Ile Ala Lys Tyr Thr Lys Gln Gly Tyr
            20                  25                  30
Glu Val Gly Ile Cys Asp Leu Thr Glu Ala Asp Leu Ser Ser Asn Gly
        35                  40                  45
Thr Ile Glu Leu Arg Lys Glu Glu Ala Lys Val Ala Ala Arg Ile Met
    50                  55                  60
Gly Val Lys Thr Arg Leu Asn Leu Ala Met Pro Asp Arg Gly Leu Tyr
65                  70                  75                  80
Met Lys Glu Glu Tyr Ile Arg Glu Ile Val Lys Val Ile Arg Thr Tyr
                85                  90                  95
Lys Pro Lys Leu Val Phe Ala Pro Tyr Tyr Glu Asp Arg His Pro Asp
            100                 105                 110
His Ala Asn Cys Ala Lys Leu Val Glu Glu Ala Ile Phe Ser Ala Gly
        115                 120                 125
Ile Arg Lys Tyr Met Pro Glu Leu Ser Pro His Arg Val Glu Ser Phe
    130                 135                 140
Tyr Asn Tyr Met Ile Asn Gly Phe His Lys Pro Asn Phe Cys Ile Asp
145                 150                 155                 160
Ile Ser Glu Tyr Leu Ser Ile Lys Val Glu Ala Leu Glu Ala Tyr Glu
                165                 170                 175
Ser Gln Phe Ser Thr Gly Ser Asp Gly Val Lys Thr Pro Leu Thr Glu
            180                 185                 190
Gly Tyr Val Glu Thr Val Ile Ala Arg Glu Lys Met Phe Gly Lys Glu
        195                 200                 205
Val Gly Val Leu Tyr Ala Glu Gly Phe Met Ser Lys Lys Pro Val Leu
    210                 215                 220
Leu His Ala Asp Leu Leu Gly Gly Cys Lys
225                 230

<110>  University of Crete
      University of Crete
      Bouriotis, Vassilis

<120>  BC1534 deacetylase

<130>  S05830

<160>  2

<170>  PatentIn version 3.3

<210>  1
<211>  750
<212>  DNA
<213>  Bacillus cereus

<400>  1
atgagtggat tacatatatt agcgtttggt gctcatgccg atgatgttga aatcggcatg      60
gctggtacca tcgctaaata tacaaagcaa gggtatgaag taggtatttg tgatttaaca     120
gaagctgatc tttcttcaaa tggaacgata gagttgagaa aagaagaagc gaaggtcgca     180
gctcgtataa tgggagtaaa aacgaggctg aatttagcga tgccagaccg tggtttgtat     240
atgaaagaag agtatatacg tgaaattgtc aaggtgatcc gtacatataa accaaaacta     300
gtttttgcgc cgtactatga agatcgccat ccagatcatg caaattgtgc aaagcttgtg     360
gaagaggcta ttttttcagc aggaatccgt aaatatatgc cggaactttc accacatcgt     420
gtggagtctt tttataatta tatgattaat ggttttcata aaccgaattt ttgtatagat     480
ataagtgagt acctctctat aaaggtggaa gcattagaag cgtatgaaag tcagttttca     540
acagggagtg atggtgttaa gacaccgtta acagaaggtt acgttgaaac tgtgattgct     600
cgtgagaaga tgtttggaaa agaagttgga gtcttgtatg ccgagggatt tatgagtaag     660
aaaccggttt tattacatgc tgatttatta gggggatgta aatgaaaacc ggttttatta     720
catgctgatt tattaggggg atgtaaatga                                      750

<210> 2
<211> 234
<212> PRT
<213> Bacillus cereus

<400> 2

Met Ser Gly Leu His Ile Leu Ala Phe Gly Ala His Ala Asp Asp Val
1               5                   10                  15
Glu Ile Gly Met Ala Gly Thr Ile Ala Lys Tyr Thr Lys Gln Gly Tyr
            20                  25                  30
Glu Val Gly Ile Cys Asp Leu Thr Glu Ala Asp Leu Ser Ser Asn Gly
        35                  40                  45
Thr Ile Glu Leu Arg Lys Glu Glu Ala Lys Val Ala Ala Arg Ile Met
    50                  55                  60
Gly Val Lys Thr Arg Leu Asn Leu Ala Met Pro Asp Arg Gly Leu Tyr
65                  70                  75                  80
Met Lys Glu Glu Tyr Ile Arg Glu Ile Val Lys Val Ile Arg Thr Tyr
                85                  90                  95
Lys Pro Lys Leu Val Phe Ala Pro Tyr Tyr Glu Asp Arg His Pro Asp
            100                 105                 110
His Ala Asn Cys Ala Lys Leu Val Glu Glu Ala Ile Phe Ser Ala Gly
        115                 120                 125
Ile Arg Lys Tyr Met Pro Glu Leu Ser Pro His Arg Val Glu Ser Phe
    130                 135                 140
Tyr Asn Tyr Met Ile Asn Gly Phe His Lys Pro Asn Phe Cys Ile Asp
145                 150                 155                 160
Ile Ser Glu Tyr Leu Ser Ile Lys Val Glu Ala Leu Glu Ala Tyr Glu
                165                 170                 175
Ser Gln Phe Ser Thr Gly Ser Asp Gly Val Lys Thr Pro Leu Thr Glu
            180                 185                 190
Gly Tyr Val Glu Thr Val Ile Ala Arg Glu Lys Met Phe Gly Lys Glu
        195                 200                 205
Val Gly Val Leu Tyr Ala Glu Gly Phe Met Ser Lys Lys Pro Val Leu
    210                 215                 220
Leu His Ala Asp Leu Leu Gly Gly Cys Lys
225                 230